# EUROPÄISCHE PATENTSCHRIFT

(11) **EP 1 977 677 B1**
(45) Veröffentlichungstag und Bekanntmachung des Hinweises auf die Patenterteilung: **19.08.2009**
(21) Anmeldenummer: 07007041.2
(22) Anmeldetag: 04.04.2007
(51) Int. Cl.: A61B 1/005

(54) **Endoskopisches Instrument**
Endoscopic instrument
Instrument endoscopique

(43) Veröffentlichungstag der Anmeldung: 08.10.2008
(73) Patentinhaber: Richard Wolf GmbH, 75438 Knittlingen (DE)
(72) Erfinder: Körner, Eberhard, Dipl.-Ing., 75438 Knittlingen (DE)
(74) Vertreter: Hemmer, Arnd

(56) Entgegenhaltungen:
- EP-A1- 0 535 847
- DE-A1- 19 534 112
- DE-A1- 19 928 272
- US-B1- 6 364 828

## Beschreibung

Die Erfindung betrifft ein endoskopisches Instrument mit einem flexibel ausgebildeten Rohrschaft.

Beispielsweise aus DE 195 34 112 A1 ist ein endoskopisches Instrument bekannt, welches einen abwinkelbaren bzw. flexiblen Rohrschaft aufweist. Dieser Rohrschaft besteht aus mehreren Rohrabschnitten, welche gelenkig miteinander in Eingriff sind. Nachteilig bei diesem Instrument ist jedoch, dass die einzelnen Rohrabschnitte vorgespannt werden müssen, um wesentliche Kräfte übertragen zu können. Dazu ist bei diesem bekannten Instrument in der Wandung des Rohrschaftes eine Nut vorgesehen, in welcher ein Zugelement formschlüssig eingreift.

US 6,364,828 B1 offenbart ein invasives medizinisches Instrument, welches flexibel ausgebildet ist. Der flexible Schaft ist aus einer Vielzahl gelenkig miteinander verbundener Rohrabschnitte gebildet. Um einen guten elektrischen Kontakt zwischen zwei aneinander angrenzenden Rohrabschnitten sicherzustellen, ist in den Gelenken jeweils ein Federelement ausgebildet, welches einstückig mit dem ersten Rohrabschnitt ausgebildet ist und federnd gegen den angrenzenden zweiten Rohrabschnitt drückt, um dort einen elektrischen Kontakt sicherzustellen. Die einzelnen Rohrabschnitte sind jedoch vollständig voneinander getrennt ausgebildet.

Im Hinblick auf diesen Stand der Technik ist es Aufgabe der Erfindung, ein endoskopisches Instrument mit einem flexibel ausgebildeten Rohrschaft zu schaffen, bei welchem der Rohrschaft eine möglichst große Flexibilität aufweist und darüber hinaus größere Kräfte übertragen werden können.

Diese Aufgabe wird durch ein endoskopisches Instrument mit den im Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße endoskopische Instrument weist einen flexibel ausgebildeten Rohrschaft auf, welcher aus mehreren Rohrabschnitten gebildet ist. Die Rohrabschnitte sind axial hintereinanderliegend angeordnet und jeweils gelenkig miteinander verbunden, so dass die einzelnen Rohrabschnitte gelenkig gegeneinander verschwenken können.

Zusätzlich zu den gelenkigen Verbindungen sind die einzelnen Rohrabschnitte jeweils über zumindest einen federnden Verbindungssteg miteinander verbunden. Der Verbindungssteg bzw. die Verbindungsstege verbinden jeweils benachbarte Rohrabschnitte in der Weise, dass der Verbindungssteg in axialer Richtung des Rohrschaftes federnd ausgebildet ist. Die Verbindungsstege sind mit den angrenzenden Rohrabschnitten vorzugsweise körperlich, insbesondere stoffschlüssig verbunden. Die Anordnung des zusätzlichen Verbindungssteges hat den Vorteil, dass gegenüber der rein gelenkigen Verbindung eine festere Verbindung der einzelnen Rohrabschnitte geschaffen wird, welche eine größere Kraftübertragung zulässt. Gleichzeitig gewährleistet die federnde Gestaltung des Verbindungssteges eine ausreichende Beweglichkeit zwischen den einzelnen Rohrabschnitten, so dass eine große Flexibilität des Rohrschaftes aufrechterhalten wird.

Die Verbindungsstege sorgen dafür, dass neben der gelenkigen Verbindung eine feste Verbindung zwischen den einzelnen Rohrabschnitten besteht, so dass diese zusammengehalten werden. So kann der Verbindungssteg besonders bevorzugt quer zur Axialrichtung wirkende Kräfte übertragen, welche von den gelenkigen Verbindungen nicht übertragen werden können.

Die Verbindungsstege sind einstückig mit den angrenzenden Rohrabschnitten ausgebildet. Dies ermöglicht eine feste Verbindung aller Rohrabschnitte, ohne dass komplizierte Montagevorgänge erforderlich wären. Darüber hinaus kann die Anbindung des Verbindungssteges an die Rohrabschnitte äußerst schlank bzw. dünn gestaltet werden, so dass das freie Lumen im Inneren des Rohrschaftes möglichst groß ausgebildet werden kann.

Die Verbindungsstege sind weiter bevorzugt Teil der Umfangswandungen der Rohrabschnitte. D. h. die Verbindungsstege sind in den Umfangswandungen der Rohrabschnitte bzw. des Rohrschaftes selber ausgebildet. Diese Ausgestaltung hat den Vorteil, dass weder der Außendurchmesser des Rohrschaftes vergrößert wird, noch das freie Lumen im Inneren des Rohrschaftes durch die Verbindungsstege verkleinert wird. Die Verbindungsstege liegen aufgrund der Ausbildung in der Umfangswandung in Axialrichtung gesehen fluchtend in der Umfangswandung und kragen in radialer Richtung vorzugsweise weder nach innen noch nach außen aus der Umfangswandung aus.

Weiter bevorzugt wirkt der zumindest eine Verbindungssteg in axialer Richtung des Rohrschaftes als Zugfeder. Ein solcher Verbindungssteg ist bevorzugt an der Seite des Rohrschaftes angeordnet, welche beim Abwinkeln des Rohrschaftes verlängert bzw. gedehnt wird. Dies bewirkt dann, dass der Verbindungssteg beim Abwinkeln des Rohrschaftes gedehnt wird, so dass aufgrund der Wirkung als Zugfeder Rückstellkräfte erzeugt werden, welche den Rohrschaft selbsttätig in seine gestreckte Lage zurückziehen bzw. die Bewegung zurück in die gestreckte Lage unterstützen. Es ist auch eine Ausgestaltung als Druckfeder möglich, dann wird der Verbindungssteg an der Umfangsseite angeordnet, zu der hin die Auslenkung des Rohrschaftes erfolgt, d. h. an der Umfangsseite, die gestaucht wird.

Das Auslenken des Rohrschaftes kann in bekannter Weise durch ein Zugmittel erfolgen, welches sich an einer Umfangsseite des Rohrschaftes in axialer Richtung erstreckt. Vorzugsweise ist dieses Zugmittel in eine Nut in der Umfangswandung eingelegt.

Besonders bevorzugt sind die Verbindungsstege zwischen den mehreren Rohrabschnitten alle im selben Umfangsbereich des Rohrschaftes gelegen. D. h. die einzelnen Verbindungsstege zwischen jeweils benachbarten Rohrabschnitten liegen bevorzugt in Axialrichtung alle auf einer Linie an derselben Winkelposition bezüglich der Längsachse des Rohrschaftes. Besonders bevorzugt handelt es sich dabei um die Umfangsseite bzw. Winkelposition, an welcher der Rohrschaft beim Abwinkeln am stärksten gedehnt wird, d. h. demjenigen Winkelbereich, an welchem sich die einzelnen Rohrabschnitte beim Abwinkeln am meisten voneinander entfernen.

Der Rohrschaft ist vorzugsweise in einer Ebene beweglich, wobei sich die Wirkungslinien der federnden Verbindungsstege zwischen den Rohrabschnitten in dieser Ebene erstrecken. D. h., wie vorangehend beschrieben, erstrecken sich die Verbindungsstege mit ihren Wirkungslinien vorzugsweise entlang einer Linie in axialer Richtung des Rohrschaftes, wobei diese eine Linie in der Bewegungsebene gelegen ist, in welcher der Rohrschaft abgewinkelt wird.

Gemäß einer weiteren bevorzugten Ausführungsform sind jeweils zwei zueinander benachbarte Rohrabschnitte über in der Umfangswandung der Rohrabschnitte ausgebildete scharnierartige Gelenke formschlüssig miteinander in Eingriff. Diese Gelenke ermöglichen eine große Beweglichkeit der Rohrabschnitte zueinander in der Knick- bzw. Auslenkrichtung des Rohrschaftes. Durch die Ausgestaltung der Gelenke innerhalb der Umfangswandung, in der Weise, dass die Gelenke vorzugsweise in radialer Richtung weder nach außen noch nach innen von der Umfangswandung auskragen, wird dabei der Außendurchmesser des Instrumentes gering und gleichzeitig das freie Lumen im Inneren möglichst groß gehalten. Diese scharnierartigen Gelenke können beispielsweise wie aus DE 195 34 112 A1 bekannt ausgebildet sein.

Der Verbindungssteg zwischen zwei benachbarten Rohrabschnitten verläuft vorzugsweise mäanderförmig bezüglich seiner Wirkungslinie.

Durch diese S-förmige bzw. mäanderförmige Gestalt wird die Federwirkung in Richtung der Wirkungslinie erreicht. Dabei wird die Federwirkung im Wesentlichen nicht durch Dehnung des Materials in Längsrichtung des Verbindungssteges, sondern in erster Linie durch elastische Biegung in der S- bzw. mäanderförmigen Gestalt des Verbindungssteges erreicht. Die einzelnen Stege bzw. Schenkel werden dabei um ihre Verbindungsbereiche auseinandergebogen. Bei Ausgestaltung als Druckfeder werden die Schenkel entsprechend zusammengebogen, so dass es zu einer Stauchung des Verbindungssteges kommt. Auf diese Weise kann eine große Längenänderung, insbesondere Längung bzw. Dehnung des Verbindungssteges in axialer Richtung des Rohrschaftes beim Auslenken des Rohrschaftes erreicht werden. Ferner wird eine große Stabilität in seitlicher Richtung, d. h. radialer Richtung gewährleistet.

Gemäß einer weiteren möglichen Ausführungsform der Erfindung sind zwischen zwei zueinander benachbarten Rohrabschnitten jeweils zwei federnde Verbindungsstege in diametral entgegengesetzten Umfangsbereichen angeordnet, welche die Rohrabschnitte miteinander verbinden, wobei vorzugsweise einer der Verbindungsstege als Zug- und der andere als Druckfeder in Richtung der Längsachse des Rohrschaftes wirkt. Beide Verbindungsstege sind dabei vorzugsweise so angeordnet, dass die Wirkungslinien ihrer Federwirkung in der Schwenk- bzw. Knickebene des Rohrschaftes liegen. Dabei ist der als Zugfeder wirkende Verbindungssteg an der Seite des Rohrschaftes angeordnet, welcher beim Abwinkeln bzw. Auslenken am größten gedehnt wird, während der als Druckfeder wirkende Verbindungssteg an der diametral entgegengesetzten Seite des Rohrschaftes angeordnet ist, zu der der Rohrschaft hin ausgelenkt wird. Dies ist die Seite, welche beim Auslenken am stärksten gestaucht wird. So wird der als Druckfeder wirkende Verbindungssteg beim Auslenken gestaucht, während der als Zugfeder wirkende Verbindungssteg gedehnt wird. Auf diese Weise wird eine größere Rückstellkraft erzeugt, welche die Rohrabschnitte wieder in ihre gestreckte Ausgangslage zurückbewegt bzw. eine solche Rückstellbewegung unterstützt. Ferner wird durch die Anordnung von zwei Verbindungsstegen eine größere Stabilität zwischen den beiden Rohrabschnitten, insbesondere in Durchmesserrichtung erreicht, so dass insgesamt mit dem Rohrschaft eine größere Kraftübertragung möglich ist.

Gemäß einer weiteren bevorzugten Ausführungsform weisen die Verbindungsstege zwischen unterschiedlichen Paaren von zueinander benachbarten Rohrabschnitten unterschiedliche Federeigenschaften auf. Über die Länge des Rohrschaftes gesehen sind mehrere bzw. eine Vielzahl von Rohrabschnitten hintereinanderliegend angeordnet, welche jeweils über zumindest einen Verbindungssteg miteinander verbunden sind. Gemäß dieser bevorzugten Ausführungsform sind diese Verbindungsstege nicht alle gleich, insbesondere nicht mit den gleichen Federeigenschaften ausgebildet. So kann ein Rohrschaft geschaffen werden, welcher über seine Länge unterschiedliche Steifigkeiten bzw. flexible Eigenschaften aufweist. Es können Bereiche vorgesehen werden, welche steifer ausgebildet sind als andere Bereiche. Ferner können so Bereiche geschaffen werden, in welchen beim Auslenken bzw. Abwinkeln des Rohrschaftes dieser stärker geknickt wird als in anderen Bereichen. So kann das Instrument von seinen flexiblen Eigenschaften her optimal an den gewünschten Einsatzzweck angepasst werden.

Besonders bevorzugt sind die Verbindungsstege in den Umfangswandungen durch Einbringen von Trennfugen in die Umfangswandungen der Rohrabschnitte ausgebildet. So ist es möglich, die Verbindungsstege einstückig mit den Rohrabschnitten auszubilden, indem in die Wandung des Rohres, aus dem die Rohrabschnitte gebildet sind, entsprechende Einschnitte eingebracht werden, welche die Verbindungsstege ausformen. So werden die Verbindungsstege direkt in der Umfangswandung ausgebildet. Je nach Form der Einschnitte ist dabei eine sehr flexible Formgebung für die Verbindungsstege möglich. Auch die Gelenke zwischen den einzelnen Rohrabschnitten und die zwischen den Rohrabschnitten gelegenen Trennfugen werden vorzugsweise in ein durchgehendes Rohr geschnitten. So können alle Rohrabschnitte, die diese verbindenden Gelenke sowie Verbindungsstege direkt in ein einstückiges durchgehendes Rohr eingeschnitten werden. Dieses Rohr kann sich ohne Einschnitte ferner einstückig weiter proximalwärts erstrecken, so dass ein durchgehender Rohrschaft des Instrumentes geschaffen werden kann, welcher am distalen Ende flexibel bzw. auslenkbar ist und am proximalen Ende starr ausgebildet ist. Die Trennfugen bzw. Einschnitte können beispielsweise durch Laserstrahl- oder Elektrodenstrahlschneiden ausgebildet werden.

Weiter bevorzugt sind zwischen den einzelnen Rohrabschnitten Trennfugen ausgebildet, welche jeweils in einem, vorzugsweise alle Trennfugen im selben, Umfangsbereich des Rohrschaftes eine in Richtung der Längsachse des Rohrschaftes größere Breite aufweisen als in den anderen Umfangsbereichen und insbesondere als in einem diametral entgegengesetzten Umfangsbereich. D. h. in dem Bereich, zu welchem der Rohrschaft hin ausgelenkt bzw. abgewinkelt werden soll, werden die Trennfugen breiter ausgebildet, so dass dort die einzelnen Rohrabschnitte in gestrecktem Zustand des Rohrschaftes weiter voneinander entfernt sind. Im abgewinkelten Zustand des Rohrschaftes werden in diesem Bereich die einzelnen Rohrsegmente aufeinander zu bewegt, so dass die Trennfugen im vollständig abgewinkelten Zustand in diesem Bereich vorzugsweise nahezu vollständig geschlossen sind, d. h. die einzelnen Rohrabschnitte aneinander anliegen. Auf der entgegengesetzten Umfangsseite bewegen sich gleichzeitig die Rohrabschnitte auseinander, so dass beim Abwinkeln hier die Trennfugen erweitert werden. D. h. in diesem Umfangsbereich des Rohrschaftes, in welchem die größte Streckung auftritt, entfernen sich die einzelnen Rohrabschnitte voneinander, welche in der gestreckten Lage nahezu vollständig aneinander anliegen. In diesem Bereich ist vorzugsweise auch der elastische bzw. federnde Verbindungssteg angeordnet, welcher beim Abwinkeln gestreckt wird.

Die sich erweiternden Trennfugen sind vorzugsweise so ausgebildet, dass die Trennfugen sich, ausgehend von den zwei diametral entgegengesetzt angeordneten Gelenkpunkten, zu dem Umfangsbereich, welcher genau zwischen diesen Gelenkpunkten liegt, erweitern. An der entgegengesetzten Umfangsseite ist die Trennfuge nur sehr schmal ausgebildet, so dass hier die Rohrabschnitte im gestreckten Zustand des Rohrschaftes im Wesentlichen aneinander anliegen.

Nachfolgend wird die Erfindung beispielhaft anhand der beigefügten Figuren beschrieben. In diesen zeigt:
- Fig. 1: Eine Seitenansicht eines Rohrschaftes für ein endoskopisches Instrument gemäß einer ersten Ausführungsform der Erfindung,
- Fig. 2: eine vereinfachte Seitenansicht des Rohrschaftes gemäß Fig. 1,
- Fig. 3: eine vergrößerte Detailansicht des Ausschnitts III in Fig. 2,
- Fig. 4: eine Schnittansicht entlang Linie IV-IV in Fig. 3,
- Fig. 5: eine abgewinkelte Darstellung eines Rohrschaftes für ein endoskopisches Instrument gemäß einer zweiten Ausführungsform der Erfindung,
- Fig. 6: eine vergrößerte Darstellung der gelenkigen Verbindung zweier Rohrabschnitte gemäß der Ausführungsform in Fig. 5,
- Fig. 7: eine Seitenansicht des Rohrschaftes gemäß Fig. 5 von der Seite der Verbindungsstege her gesehen,
- Fig. 8: eine um 90° gedrehte Seitenansicht des Rohrschaftes gemäß Fig. 7,
- Fig. 9: eine Schnittansicht des Rohrschaftes gemäß Fig. 8,
- Fig. 10: eine Ansicht des Rohrschaftes gemäß Fig. 5 bis 9 im abgewinkelten Zustand,
- Fig. 11: eine Schnittansicht des abgewinkelten Rohrschaftes gemäß Fig. 10,
- Fig. 12: eine Seitenansicht eines Rohrschaftes für ein endoskopisches Instrument gemäß einer dritten Ausführungsform der Erfindung,
- Fig. 13: eine um 90° gedrehte Seitenansicht des Rohrschaftes gemäß Fig. 12,
- Fig. 14: eine gegenüber Fig. 13 nochmals um 90° gedrehte Seitenansicht des Rohrschaftes gemäß der Fig. 12 und 13 und
- Fig. 15: einen Rohrschaft gemäß einer weiteren Ausführungsform der Erfindung.

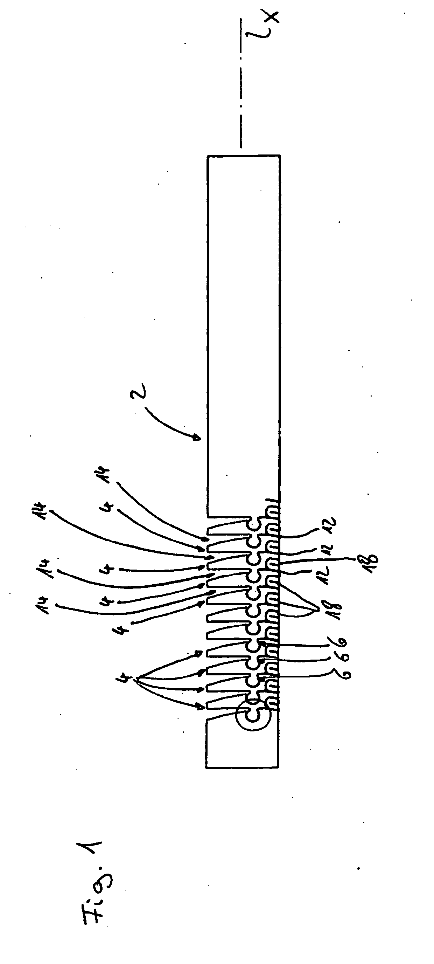

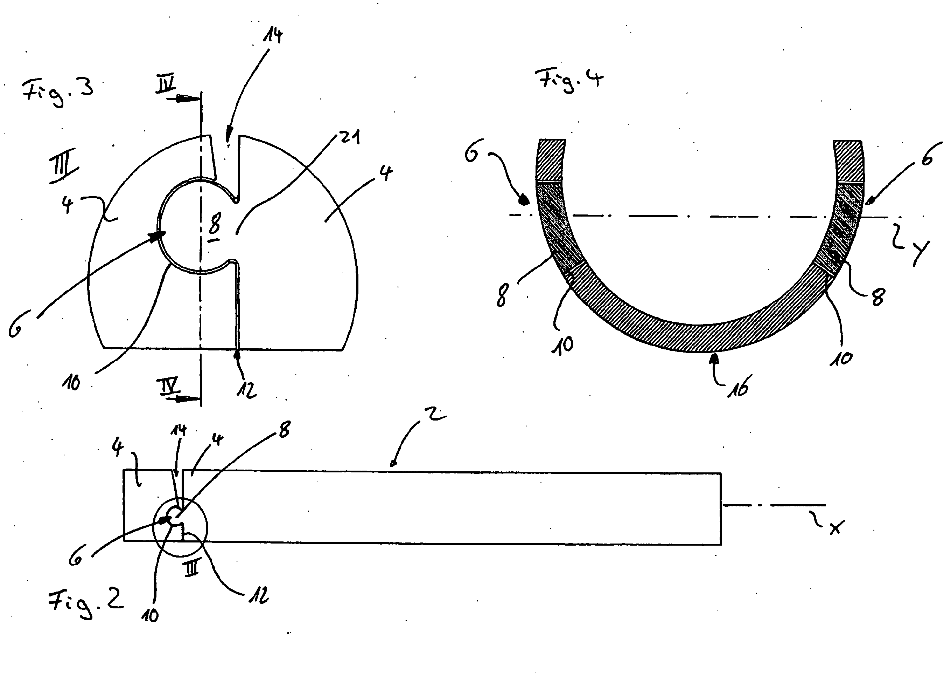

Anhand der Fig. 1 bis 4 wird der grundsätzliche Aufbau eines endoskopischen Instrumentes bzw. eines Rohrschaftes für ein solches Instrument gemäß einer ersten bevorzugten Ausführungsform der Erfindung erläutert. Zu beachten ist, dass in Fig. 1 und 2 jeweils nur der Rohrschaft des Instrumentes schematisch dargestellt ist, wobei in Fig. 1 nur einige der Vielzahl von Rohrabschnitten 4 des Rohrschaftes 2 dargestellt sind, aus welchen der flexible Teil des Rohrschaftes 2 gebildet wird. Es ist zu verstehen, dass hier noch weitere Rohrabschnitte entsprechender Ausgestaltung in dem Rohrschaft 2 ausgebildet sein können. Entsprechend ist in Fig. 2 nur eine einzige Verbindung zwischen zwei Rohrabschnitten 4 zur Veranschaulichung der Ausgestaltung dieser Verbindung dargestellt.

Der flexible bzw. gelenkige Teil des Rohrschaftes 2 ist aus einer Mehrzahl in axialer Richtung X hintereinanderliegenden Rohrabschnitten 4 gebildet. Die Rohrabschnitte 4 sind jeweils an zwei diametral entgegengesetzt in der Umfangswandung ausgebildeten Gelenken 6 formschlüssig miteinander in Eingriff. Diese gelenkige Verbindung ist ähnlich aufgebaut, wie es beispielsweise aus DE 195 34 112 A1 oder aus DE 195 35 179 A1 bekannt ist.

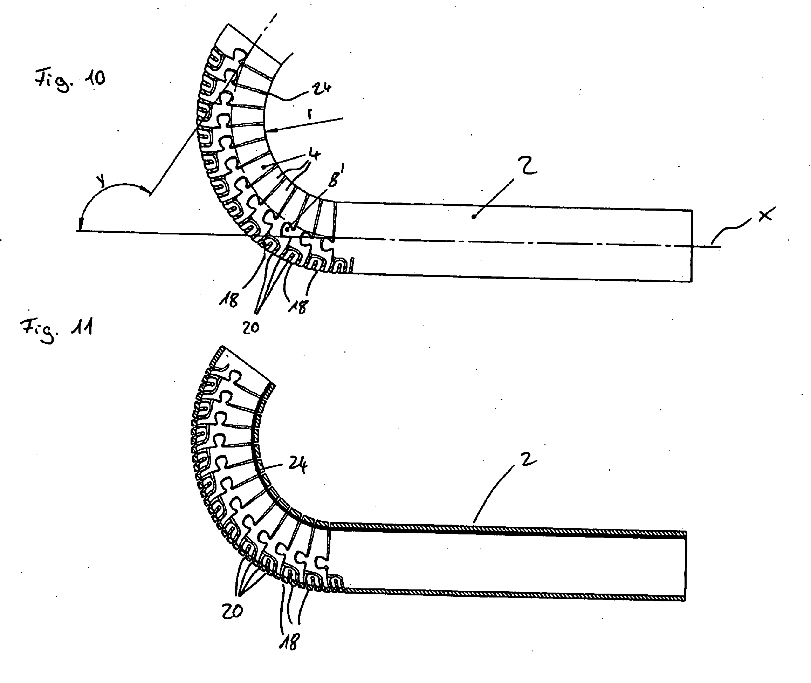

Wie in Fig. 2 und 3 deutlicher gezeigt, weisen die Gelenkverbindungen 6 jeweils einen kreisförmigen Zapfen 8 an einem Rohrabschnitt und eine korrespondierende kreisförmige Ausnehmung 10 an einem angrenzenden Rohrabschnitt 4 auf. Der Zapfen 8 greift passend und formschlüssig in die Ausnehmung 10 ein, so dass er sich in der Ausnehmung 10 drehen kann. Dazu ist die Trennfuge 12 zwischen den beiden Rohrabschnitten 4 so ausgebildet, dass an einer Seite des Umfangs ausgehend von den zwei diametral gegenüberliegenden Gelenkverbindungen 6 breitere Ausschnitte 14 ausgebildet sind. D. h. in diesem Bereich sind die benachbarten Rohrabschnitte 4 weiter voneinander beabstandet. Dabei sind die Ausschnitte 14 so geformt, dass sie sich ausgehend von den beiden Gelenkverbindungen 6 erweitern, so dass die größte Breite in Längsrichtung X im Wesentlichen an einer Winkelposition um 90° versetzt zu den beiden Gelenkverbindungen 6 am Umfang des Rohrschaftes 2 gelegen ist. Die Ausschnitte 14 ermöglichen es, dass jeweils zwei benachbarte Rohrabschnitte 4 um die Gelenkverbindungen 6 zu der Umfangsseite hin, an welcher die Ausschnitte 14 ausgebildet sind, verschwenkt werden können. Die einzelnen Rohrabschnitte 4 verschwenken jeweils gelenkig zueinander um eine Schwenkachse, welche sich durch die Mittelpunkte der Zapfen 8 quer bzw. normal zur Längsachse X erstreckt. Durch das Verschwenken der einzelnen Rohrabschnitte 4 gegeneinander wird insgesamt eine flexible Auslenkung des Rohrschaftes, wie in Fig. 10 und 11 dargestellt, erreicht. Dabei krümmt sich der Rohrschaft um einen externen fiktiven Punkt mit dem Radius r, wobei sich der Radius r mit zunehmender Krümmung verkleinert. Die maximale Krümmung mit dem maximalen Schwenkwinkel Y jedes Rohrschaftes 2 ist dann erreicht, wenn im Bereich der Ausschnitte 14 die einander gegenüberliegenden stirnseitigen Kanten der benachbarten Rohrabschnitte 4 aneinander zu liegen kommen. Gleichzeitig weitet sich die Trennfuge 12 an der diametral engegengesetzten Seite des Ausschnittes 14 auf.

Wie in der Schnittansicht in Fig. 4 zu erkennen ist, sind die Gelenkverbindungen 6 vollständig in der Wandung 16 der Rohrabschnitte 4 bzw. des Rohrschaftes 2 ausgebildet. Auf diese Weise wird der zur Verfügung stehende Querschnitt optimal ausgenutzt. Das bedeutet, dass zum einen der Außendurchmesser des Instrumentes durch die Gelenkverbindungen nicht vergrößert wird und gleichzeitig auch das freie Lumen im Inneren des Rohrschaftes 2 durch die Gelenkverbindungen 6 nicht verkleinert wird. Bei der in Fig. 1 bis 4 gezeigten Ausführungsform sind die Zapfen 8 und die Ausnehmungen 10 mit dem Verlauf der Umfangswandung 16 gekrümmt. Dies bewirkt, dass die Zapfen 8 und die Ausnehmungen 10 an ihrer radial innenliegenden Seite in der Erstreckungsrichtung der Umfangsrichtung 16 einen kleineren Durchmesser aufweisen als an der Außenseite. D. h. die Zapfen 8 sind insgesamt somit zum Inneren des Rohrschaftes 2 hin konisch ausgebildet. Auf diese Weise kann ein seitliches Verschieben in Durchmesserrichtung bzw. Richtung der Schwenkachse Y quer bzw. normal zur Längsachse X verhindert werden.

Erfindungsgemäß sind die einzelnen Rohrabschnitte 4 durch die Trennfugen 12 nicht vollständig voneinander getrennt. Vielmehr sind die Trennfugen 12 an der den Ausschnitte 14 diametral entgegengesetzten Seite so geformt, dass jeweils durchgehende Verbindungsstege 18 zwischen den einzelnen Rohrabschnitten 4 ausgebildet sind, welche benachbarte Rohrabschnitte 4 miteinander verbinden. D. h. die Rohrabschnitte 4 sind nicht vollständig voneinander getrennt, sondern werden zusätzlich zu den Gelenkverbindungen 6 durch die Verbindungsstege 18 zusammengehalten. Die Verbindungsstege 18 erstrecken sich zickzackförmig bzw. S- bzw. mäanderförmig bezüglich der Längsachse X zwischen angrenzenden Rohrabschnitten 4. Auf diese Weise wird eine Elastizität bzw. Federwirkung der Verbindungsstege 18 in Richtung der Längsachse X erreicht.

So können sich die Verbindungsstege 18 beim Auslenken bzw. Verschwenken des flexiblen Abschnittes des Rohrabschnittes 2, bei welchem sich die einzelnen Rohrabschnitte 4, wie oben beschrieben, um ihre Zapfen 8 verschwenken, dehnen. In dem Bereich zwischen den Rohrabschnitten 4, in dem die Verbindungsstege 18 angeordnet sind, entfernen sich die Rohrabschnitte 4 beim Verschwenken voneinander, während sie im Bereich des Ausschnittes 14 sich annähern. Die dabei erfolgende Dehnung der Verbindungsstege 18 erfolgt dadurch, dass in dem S-förmigen Verlauf der Verbindungsstege 18 eine Biegung im Bereich der Kurven bzw. Radien der Verbindungsstege 18 erfolgt, wobei die geraden Abschnitte bzw. Schenkel zwischen diesen Radien der Verbindungsstege 18 sich weiter voneinander entfernen. Diese elastische Verformung der Verbindungsstege 18 erzeugt eine Rückstell- bzw. Federkraft, welche für eine Rückstellung des Rohrschaftes 2 in die in Fig. 1 gezeigte gestreckte Lage sorgt, sobald die Kraft zum Auslenken gelöst wird, oder diese Rückstellbewegung zumindest erleichtert. Der gedehnte Zustand der Verbindungsstege 18 ist in den Fig. 10 und 11 zu erkennen, in welchen der Rohrschaft 2 ausgelenkt bzw. gekrümmt ist. Dort ist zu erkennen, dass sich durch die Dehnung der Verbindungsstege 18 zwischen den in Umfangsrichtung verlaufenden Schenkeln der Verbindungsstege 18 Spalte 20 bilden, während in dem gestreckten Zustand, welcher in den Fig. 1, 5, 7 und 8 gezeigt ist, diese Teile der Verbindungsstege 18 bis auf die schmalen Trennfugen 12 im Wesentlichen direkt aneinander anliegen.

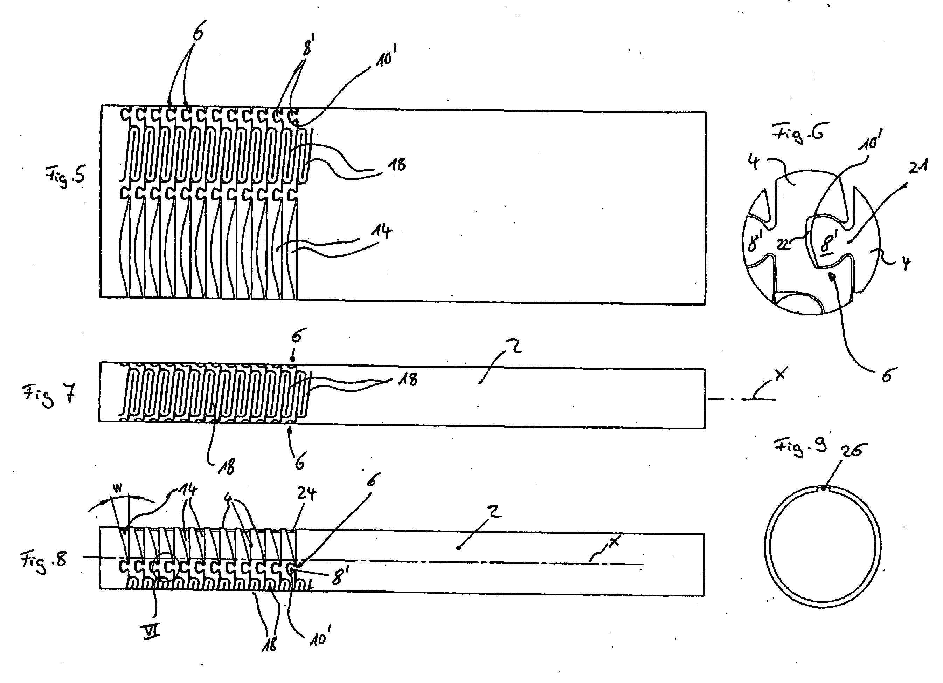

In den Fig. 5 bis 8 sowie 10 und 11 ist eine zweite Ausführungsform in der Erfindung gezeigt, welche sich von der anhand der Fig. 1 bis 4 erläuterten Ausführungsformen nur in der Ausgestaltung der Gelenkverbindungen 6 unterscheidet. Alle übrigen Details und insbesondere die Ausgestaltung der Verbindungsstege 18 sind identisch. Bei der Ausgestaltung gemäß Fig. 5 bis 8 und 10 bis 11 sind die Zapfen 8' nicht kreisförmig und die Ausnehmungen 10' entsprechend ebenfalls nicht kreisförmig ausgebildet. Die Zapfen 8' weisen eine teilkreisförmige Außenkontur an derjenigen Seite auf, an welcher sie über einen Steg 21 mit einem angrenzenden Rohrabschnitt 4 verbunden sind. In dem, dem Steg 21 abgewandten Bereich, ist der Zapfen 8' verkürzt bzw. abgeschnitten ausgebildet. Entsprechend weist die Ausnehmung 10' nur in dem an ihre Öffnung angrenzenden Bereich eine teilkreisförmige Kontur auf, der Boden der Ausnehmung 10' ist ebenfalls abgeschnitten, wobei in diesem Bereich zwischen dem Boden der Ausnehmung 10' und der Stirnseite des Zapfens 8' ein Freiraum 22 verbleibt, wie deutlich in der Ausschnittsvergrößerung von Fig. 6 zu sehen ist. Dieser Freiraum 22 ermöglicht die Bewegung des Zapfens 8' in der Ausnehmung 10', wobei die benachbarten Rohrabschnitte 4, wie anhand der Fig. 1 bis 4 erläutert, gegeneinander verschwenken, was in Fig. 10 und 11 dargestellt ist. Auch dort sind die Zapfen 8' und die Ausnehmungen 10' wie in Fig. 6 gezeigt ausgebildet. Das Verschwenken würde jedoch in gleicher Weise mit den in Fig. 1 bis 4 gezeigten Zapfen 8 funktionieren.

Die in den Figuren 5 bis 8 sowie 10 und 11 gezeigte zweite Ausführungsform der Erfindung ermöglicht aufgrund der nicht vollständig kreisförmig ausgebildeten Gelenkverbindungen in Längsrichtung X schmalere Rohrabschnitte 4, wodurch insgesamt kleinere Ablenkradien erreicht werden können.

In den Fig. 5 und 7 ist der mäanderförmige Verlauf der Verbindungsstege 18 deutlicher zu sehen. Dabei zeigt Fig. 5 eine Ansicht, in welcher die Umfangswandung des Rohrabschnittes 2 aufgeschnitten und abgewickelt ist.

Die Auslenkung bzw. Krümmung des Rohrschaftes 2 erfolgt durch ein Zugelement 24, welches sich in Längsrichtung X in einer Nut 26 in der Umfangswandung 16 des Rohrschaftes 2 und Rohrabschnitte 4 erstreckt. Dieses Zugelement 24 ist am distalen Ende des Rohrschaftes befestigt und im Übrigen relativ zu dem Rohrschaft beweglich. Wenn das Zugelement 24 proximalwärts gezogen wird, wird auf diese Weise der Rohrschaft in seinem beweglichen Bereich, welcher von den Rohrabschnitten 4 gebildet wird, ausgelenkt, wie in Fig. 10 und 11 dargestellt. In Fig. 1, 2 und 9 sowie Fig. 13 und 14 ist dieses Zugelement 24 nicht dargestellt, es ist jedoch zu verstehen, dass es dort in entsprechender Weise angeordnet wird. Die Rückstellung des Rohrschaftes 2 in seine gestreckte Lage erfolgt zunächst einmal dadurch, dass die in proximaler Richtung auf das Zugelement 24 wirkende Kraft gelöst wird. Aufgrund ihrer Elastizität sind die Verbindungsstege 18 bestrebt, sich in ihre in den Fig. 1, 5 bis 8 sowie 12 bis 14 gezeigte Ausgangslage zurückzubewegen, d. h. die Verbindungsstege 18 erzeugen hier eine Rückstellkraft bzw. einen Teil der Rückstellkraft. Zusätzlich kann das Zugelement 24 als Zug-/Druckelement ausgebildet sein, so dass es auch eine Druckkraft in distaler Richtung übertragen kann, wodurch der Rohrschaft 2 in seine gestreckte Lage mit einem Schwenkwinkel Y = 0° zurückbewegt werden kann.

Die Verbindungsstege 18 zwischen den einzelnen Rohrabschnitten 4 sorgen bei gleichbleibender Flexibilität bzw. Beweglichkeit des Rohrschaftes 2 für eine erhöhte Stabilität, welche eine größere Kraftübertragung zulässt, da sie insbesondere in seitlicher, d. h. radialer bzw. Umfangsrichtung bezüglich der Längsachse X größere Kraftübertragung zwischen den zueinander beweglichen Rohrabschnitten 4 ermöglichen.

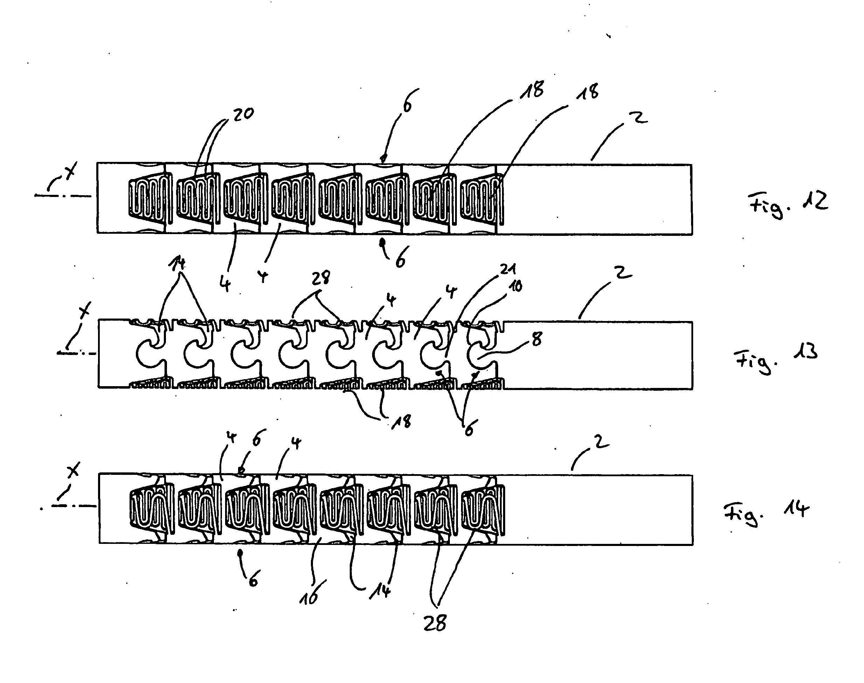

Fig. 12 bis 14 zeigen eine weitere Ausführungsform der Erfindung, bei welcher zum einen die Verbindungsstege 18 etwas anders als bei den vorangehend beschriebenen Ausführungsformen ausgestaltet sind. Die Verbindungsstege 18 weisen mehr Windungen auf und die Windungen sind auch in der in Fig. 12 bis 14 gezeigten gestreckten Ausgangslage des Rohrschaftes 2 geringfügig voneinander beabstandet, wobei sich die Spalte 20 zwischen den einzelnen Windungen bzw. sich in Umfangsrichtung erstreckenden Schenkeln der Verbindungsstege 18 bei Krümmung ähnlich zu Fig. 10 und 11 weiter voneinander entfernen werden. Darüber hinaus sind gegenüber der Ausführungsform in Fig. 1 bis 4 die Zapfen 8 und die zugehörigen Ausnehmungen 10 zwar ebenfalls von kreisförmiger Kontur ausgestaltet, weisen jedoch nahe dem Steg 21 eine hakenförmige Gestalt auf. Das Verschwenken erfolgt jedoch genauso wie anhand der Fig. 1 bis 4 beschrieben. Der Hauptunterschied zu den vorangehend beschriebenen Ausführungsformen liegt darin, dass diametral entgegengesetzt zu den Verbindungsstegen 18 in der Wandung 16 der Rohrabschnitte 4 weitere Verbindungsstege 28 zwischen benachbarte Rohrabschnitten 4 ausgebildet sind.

Die Verbindungsstege 28 sind wie die Verbindungsstege 18 in der Umfangswandung 16 des Rohrschaftes 2 selber ausgebildet und haben ebenfalls einen S- bzw. mäanderförmigen Verlauf bezüglich der Längsachse X. Allerdings sind bei den Verbindungsstegen 28 die Windungen größer und in der gezeigten Ruhelage weiter voneinander beabstandet, da die Verbindungsstege 18 eine Wirkung als Druckfeder in Längsrichtung X aufweisen. Beim Verschwenken, ähnlich zu Fig. 10 und 11 werden die Rohrabschnitte 4 um die Gelenkverbindungen 6 verschwenkt, so dass sie im Bereich der Ausschnitte 14 einander angenähert werden. Da die Verbindungsstege 28 auf der Umfangsseite, an welcher auch die Ausnehmungen 14 ihre größte Breite aufweisen, angeordnet sind, werden die Verbindungsstege 18 dabei gestaucht, d. h. ihre Windungen einander angenähert. Diese folgt durch eine Biegung im Bereich der Krümmungsradien zwischen den einzelnen Schenkeln der Verbindungsstege 28. Gleichzeitig mit der Dehnung der Verbindungsstege 18 erzeugen somit die Verbindungsstege 28 eine weitere Rückstellkraft, indem sie aufgrund ihrer Elastizität das Bestreben haben, die Rohrabschnitte 4 im Bereich der Ausschnitte 14 wieder auseinanderzudrücken. Darüber hinaus sorgen die Verbindungsstege 28, welche die benachbarten Rohrabschnitte 4 entsprechend der Anordnung der Verbindungsstege 18 einstückig miteinander verbinden, für eine zusätzliche Stabilität in Durchmesser- bzw. Umfangsrichtung.

In den gezeigten Beispielen ist eine Auslenkung bzw. Krümmung des Rohrschaftes 2 lediglich in einer Ebene in einer Richtung vorgesehen. Aus diesem Grunde sind in den gezeigten Beispielen die Gelenkverbindungen 6 alle im selben Umfangsbereich in Längsrichtung X auf einer Linie liegend angeordnet. Entsprechend sind auch die Verbindungsstege 18 bzw. 28 in Längsrichtung auf einer Linie liegend angeordnet. Es ist jedoch auch denkbar, eine Auslenkung in mehrere Richtungen zuzulassen, wobei dann einzelne Gelenkverbindungen 6 und damit entsprechend die Ausschnitte 14 sowie die Verbindungsstege 18 bzw. 28 in Umfangsrichtung versetzt, angeordnet werden können.

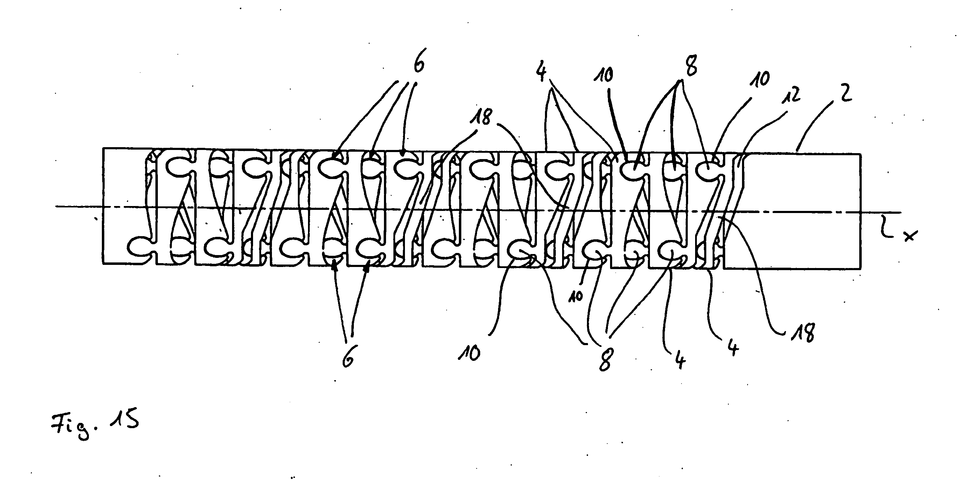

Ein Beispiel für eine solche Ausführungsform ist in Fig. 15 gezeigt. Dort sind die Rohrabschnitte 4 so miteinander verbunden, dass die Gelenkverbindungen 6, bestehend aus Zapfen 8 und Ausnehmung 10 zwischen benachbarten Rohrabschnitten 4 abwechselnd zueinander um 90° versetzt bezüglich der Längsachse X angeordnet sind. Auf diese Weise wird ein Verschwenken in zwei normal zueinander gerichteten Ebenen möglich. Dabei wechseln sich die normal zueinander gerichteten Schwenkachsen immer ab. Das heißt, in Richtung der Längsachse gesehen ist eine erste Schwenkachse zwischen einem ersten und einem zweiten Rohrabschnitt in einer ersten Richtung gerichtet, während die nächste Schwenkachse zwischen dem zweiten und einem dritten Rohrabschnitt um 90° gedreht bzw. versetzt bezüglich der Längsachse X gerichtet ist, und so weiter. Auf diese Weise wird eine Ablenkung des distalen Endes des Rohrschaftes in beliebiger Richtung möglich. Anstatt immer abwechselnd um 90° zueinander versetzte Schwenkachsen anzuordnen, ist es auch möglich, mehrere Gruppen von Rohrabschnitten zu bilden, wobei die einzelnen Gruppen jeweils aus mehreren Rohrabschnitten bestehen, zwischen denen die Schwenkachsen in derselben Richtung gerichtet sind. Die einzelnen Gruppen unterscheiden sich dann dadurch, dass die Schwenkachsen der unterschiedlichen Gruppen im Winkel zueinander, vorzugsweise in einem Winkel von 90° zueinander gerichtet sind.

Ferner ist es auch möglich, eine weitere Ausnehmung 14 diametral entgegengesetzt im Bereich der Verbindungsstege 18 anzuordnen, so dass ein Verschwenken auch in die entgegengesetzte Richtung möglich ist. Dann würde der Verbindungssteg 18 entsprechend dem Verbindungssteg 28 als Druckfeder wirken.

Auch sind in den gezeigten Beispielen über die Länge des Rohrschaftes 2 sämtliche Rohrabschnitte 4 und insbesondere sämtliche Verbindungsstege 18 bzw. 28 identisch ausgebildet. Es ist jedoch denkbar, einzelne Verbindungsstege 18 bzw. 28 über die Länge des Rohrschaftes 2 unterschiedlich auszubilden, um unterschiedliche Steifigkeiten, Federkräfte und/oder Gelenkeigenschaften zwischen einzelnen Rohrabschnitten 4 bereitzustellen. So kann das Instrument durch Gestaltung seines Rohrschaftes 2 an den gewünschten Einsatzzweck optimal angepasst werden. Auch ist zu verstehen, dass die Gelenkverbindungen 6 auch auf andere Weise, insbesondere anders geformt, ausgestaltet werden können.

Der Rohrschaft 2 mit den Ausschnitten 14, den Gelenkverbindungen 6 und den Verbindungsstegen 18 bzw. 28 wird aus einem Rohr gefertigt, indem die Trennfugen 12 sowie die Ausnehmungen und Fugen, welche die Verbindungsstege 18 bilden, in das Rohr eingeschnitten werden. Dies kann beispielsweise durch Laserstrahl- bzw. Elektronenstrahlschneiden erfolgen. Auf diese Weise ist es sehr einfach möglich, komplexe Formgebungen in die Wandung 16 des Rohrschaftes 2 einzubringen und die einzelnen Rohrabschnitte 4 einstückig auszugestalten und gleichzeitig die Beweglichkeit um die Gelenkverbindungen zu gewährleisten.

### Bezugszeichenliste

- 2: Rohrschaft
- 4: Rohrabschnitte
- 6: Gelenkverbindung
- 8, 8': Zapfen
- 10, 10': Ausnehmung
- 12: Trennfuge
- 14: Ausschnitte
- 16: Wandung
- 18: Verbindungsstege
- 20: Spalt
- 21: Stege
- 22: Freiraum
- 24: Zugelement
- 26: Nut
- 28: Verbindungsstege
- X: Längsachse
- Y: Schwenkachse
- r: Schwenkwinkel
- r: Schwenkradius

## Patentansprüche

1. Endoskopisches Instrument mit einem flexibel ausgebildeten Rohrschaft (2), bei welchem der Rohrschaft (2) aus mehreren gelenkig miteinander verbundenen Rohrabschnitten (4) gebildet ist,
**dadurch gekennzeichnet, dass**
jeweils zwei zueinander benachbarte Rohrabschnitte (4) zusätzlich zu der gelenkigen Verbindung (6) über zumindest einen in axialer Richtung (X) des Rohrschaftes (2) federnden Verbindungssteg (18) miteinander verbunden sind, welcher in der Weise einstückig mit den angrenzenden Rohrabschnitten (4) ausgebildet ist, dass die angrenzenden Rohrabschnitte nicht vollständig durch eine Trennfuge voneinander getrennt sind.

2. Endoskopisches Instrument nach Anspruch 1, **dadurch gekennzeichnet, dass** der Verbindungssteg (18) Teil der Umfangswandungen (16) der Rohrabschnitte (4) ist.

3. Endoskopisches Instrument nach einem der vorangehenden Ansprüche, **dadurch gekennzeichnet, dass** der Verbindungssteg (18) in axialer Richtung (X) des Rohrschaftes (2) als Zugfeder wirkt.

4. Endoskopisches Instrument nach einem der vorangehenden Ansprüche, **dadurch gekennzeichnet, dass** die Verbindungsstege (18) zwischen den mehreren Rohrabschnitten (2) alle im selben Umfangsbereich des Rohrschaftes (2) liegen.

5. Endoskopisches Instrument nach einem der vorangehenden Ansprüche, **dadurch gekennzeichnet, dass** der Rohrschaft (2) in einer Ebene beweglich ist, wobei sich die Wirkungslinien der federnden Verbindungsstege (18) zwischen den Rohrabschnitten (4) in dieser Ebene erstrecken.

6. Endoskopisches Instrument nach einem der vorangehenden Ansprüche, **dadurch gekennzeichnet, dass** jeweils zwei benachbarte Rohrabschnitte (4) über in der Umfangswandung (16) der Rohrabschnitte (4) ausgebildete scharnierartige Gelenke (6) formschlüssig miteinander in Eingriff sind.

7. Endoskopisches Instrument nach einem der vorangehenden Ansprüche, **dadurch gekennzeichnet, dass** der Verbindungssteg (18) mäanderförmig bezüglich seiner Wirkungslinie verläuft.

8. Endoskopisches Instrument nach einem der vorangehenden Ansprüche, **dadurch gekennzeichnet, dass** zwei zueinander benachbarte Rohrabschnitte (4) jeweils über zwei in diametral entgegengesetzten Umfangsbereichen angeordnete federnde Verbindungsstege (18, 28) miteinander verbunden sind, von denen vorzugsweise einer als Zug- (18) und der andere als Druckfeder (28) in Richtung der Längsachse (X) des Rohrschaftes (2) wirkt.

9. Endoskopisches Instrument nach einem der vorangehenden Ansprüche, **dadurch gekennzeichnet, dass** die Verbindungsstege (18, 28) zwischen unterschiedlichen Paaren von zueinander benachbarten Rohrabschnitten (4) unterschiedliche Federeigenschaften aufweisen.

10. Endoskopisches Instrument nach einem der vorangehenden Ansprüche, **dadurch gekennzeichnet, dass** die Verbindungsstege (18, 28) in den Umfangswandungen (10) durch Einbringen von Trennfugen in die Umfangswandungen der Rohrabschnitte (4) ausgebildet sind.

11. Endoskopisches Instrument nach einem der vorangehenden Ansprüche, **dadurch gekennzeichnet, dass** zwischen den einzelnen Rohrabschnitten (4) Trennfugen (12) ausgebildet sind, welche jeweils in einem, vorzugsweise alle Trennfugen (12) im selben, Umfangsbereich(14) des Rohrschaftes (2) eine in Richtung der Längsachse (X) des Rohrschaftes (2) größere Breite aufweisen als in den anderen Umfangsbereichen und insbesondere als in einem diametral entgegengesetzten Umfangsbereich.

## Claims

1. An endoscopic instrument with a flexibly designed tube shank (2), with which the tube shank (2) is formed of several tube sections (4) which are articulately connected to one another, **characterized in that** in each case two tube sections (4) adjacent to one another, additionally to the articulated connection (6), are connected to one another via at least one connection web (18) which is resilient in the axial direction (X) of the tube shank (2), said the connection web (18) being designed as one piece with the adjacent tube sections (4), in a manner such that the adjacent tube sections are not completely separated from one another by a separating gap.

2. An endoscopic instrument according to claim 1, wherein the connection web (18) is part of the peripheral walls (16) of the tube sections (4).

3. An endoscopic instrument according to one of the preceding claims, wherein the connection web (18) acts as a tension spring in the axial direction (X) of the tube shank (2).

4. An endoscopic instrument according to one of the preceding claims, wherein the connection webs (18) between the several tube sections (2) all lie in the same peripheral region of the tube shank (2).

5. An endoscopic instrument according to one of the preceding claims, wherein the tube shank (2) is movable in a plane, wherein the lines of action of the resilient connection webs (18) extend between the tube sections (4) in this plane.

6. An endoscopic instrument according to one of the preceding claims, wherein in each case two adjacent tube sections (4) are engaged with one another with a positive fit, via hinge-like joints (6) formed in the peripheral wall (16) of the tube sections (4).

7. An endoscopic instrument according to one of the preceding claims, wherein the connection web (18) runs in a meandering manner with respect to its line of action.

8. An endoscopic instrument according to one of the preceding claims, wherein two tube sections (4) which are adjacent to one another are connected to one another in each case via two resilient connection webs (18, 28) which are arranged in diametrically opposite peripheral regions, and of which preferably one acts as a tension spring (18) and the other as a compression spring (28), in the direction of the longitudinal axis (X) of the tube shank (2).

9. An endoscopic instrument according to one of the preceding claims, wherein the connection webs (18, 28) between different pairs of tube sections (4) which are adjacent to one another, have different spring characteristics.

10. An endoscopic instrument according to one of the preceding claims, wherein the connection webs (18, 28) in the peripheral walls (10) are formed by introducing separating gaps into the peripheral walls of the tube sections (4).

11. An endoscopic instrument according to one of the preceding claims, wherein separating gaps (12) are formed between the individual tube sections (4), which in each case in one separating gap, preferably all separating gaps (12) in the same peripheral region (14) of the tube shank (2), have a width which is wider in the direction of the longitudinal axis (X) of the tube shank (2), than in the other peripheral regions and in particular than in a diametrically opposite peripheral region.

## Revendications

1. Instrument endoscopique comportant une tige tubulaire (2) à structure souple, dans lequel la tige tubulaire (2) est formée de plusieurs segments tubulaires (4), reliés les uns aux autres de manière articulée, les segments tubulaires de chaque paire de segments tubulaires adjacents (4) étant, outre par le raccord articulé (6), reliés entre eux par au moins une traverse de liaison (18), ayant un effet élastique dans la direction axiale (X) de la tige tubulaire (2), traverse qui forme un seul tenant avec les segments tubulaires adjacents (4), **caractérisé en ce que** les segments tubulaires adjacents ne sont pas complètement séparés l'un de l'autre par un joint de séparation.

2. Instrument endoscopique selon la revendication 1, **caractérisé en ce que** la traverse de liaison (18) est une partie des parois périphériques (16) des segments tubulaires (4).

3. Instrument endoscopique selon l'une des revendications précédentes, **caractérisé en ce que** la traverse de liaison (18) agit comme un ressort de traction dans la direction axiale (X) de la tige tubulaire (2).

4. Instrument endoscopique selon l'une des revendications précédentes, **caractérisé en ce que** les traverses de liaison (18) sont situées entre les multiples segments tubulaires (2), toutes dans la même zone périphérique de la tige tubulaire (2).

5. Instrument endoscopique selon l'une des revendications précédentes, **caractérisé en ce que** la tige tubulaire (2) est mobile dans un plan, les lignes d'action des traverses de liaison (18) à effet élastique s'étendant entre les segments tubulaires (4) dans ce plan.

6. Instrument endoscopique selon l'une des revendications précédentes, **caractérisé en ce que** les segments tubulaires de chaque paire de segments tubulaires adjacents (4) sont en prise l'un avec l'autre par une liaison mécanique, par l'intermédiaire d'articulations (6) à charnière, conçues dans la paroi périphérique (16) des segments tubulaires (4).

7. Instrument endoscopique selon l'une des revendications précédentes, **caractérisé en ce que** la traverse de liaison (18) court en méandres par rapport à sa ligne d'action.

8. Instrument endoscopique selon l'une des revendications précédentes, **caractérisé en ce que** les segments tubulaires de chaque paire de segments tubulaires adjacents (4) sont reliés l'un à l'autre par l'intermédiaire de deux traverses de liaison (18, 28) à effet élastique, disposées dans des zones périphériques diamétralement opposées, traverses dont l'une agit de préférence comme un ressort de traction (18) et l'autre comme un ressort de compression (28) dans la direction de l'axe longitudinal (X) de la tige tubulaire (2).

9. Instrument endoscopique selon l'une des revendications précédentes, **caractérisé en ce que** les traverses de liaison (18, 28) présentent, entre des paires différentes de segments tubulaires adjacents (4), des propriétés élastiques différentes.

10. Instrument endoscopique selon l'une des revendications précédentes, **caractérisé en ce que** les traverses de liaison (18, 28) sont conçues dans les parois périphériques (10), par insertion de oints de séparation dans les parois périphériques des segments tubulaires (4).

11. Instrument endoscopique selon l'une des revendications précédentes, **caractérisé en ce que** des joints de séparation (12) sont formés entre les segments tubulaires individuels (4), joints de séparation (12) qui pour chacun, et de préférence tous, dans une et même zone périphérique (14) de la tige tubulaire (2), présentent une largeur qui, dans la direction de l'axe longitudinal (X) de la tige tubulaire (2), est plus grande que dans les autres zones périphériques et en particulier que dans la zone périphérique diamétralement opposée.
